## Europäisches Patentamt

(19) **European Patent Office**

**Office européen des brevets**

(11) Veröffentlichungsnummer : **0 265 795 B1**

(12) **EUROPÄISCHE PATENTSCHRIFT**

(45) Veröffentlichungstag der Patentschrift :
05.06.91 Patentblatt 91/23

(51) Int. Cl.$^5$ : **A61M 1/16**

(21) Anmeldenummer : **87115158.5**

(22) Anmeldetag : **16.10.87**

(54) **Vorrichtung zur Behandlung von Blut im extrakorporalen Kreislauf.**

(30) Priorität : 30.10.86 DE 3636995

(43) Veröffentlichungstag der Anmeldung :
**04.05.88 Patentblatt 88/18**

(45) Bekanntmachung des Hinweises auf die
Patenterteilung :
**05.06.91 Patentblatt 91/23**

(84) Benannte Vertragsstaaten :
**DE ES FR GB IT**

(56) Entgegenhaltungen :
**EP-A- 0 122 604**

(56) Entgegenhaltungen :
**TRANSACTION AMERICAN SOCIETY FOR AR-
TIFICIAL INTERNAL ORGANS, Band 28, 1982,
Seiten 523-527; Q. MAGGIORE et al.: "Blood
temperature and vascular stability during hemodialysis and hemofiltration"**

(73) Patentinhaber : **Fresenius AG
Gluckensteinweg 5
W-6380 Bad Homburg v.d.H. (DE)**

(72) Erfinder : **Polaschegg, Hans-Dietrich, Dr.
Grünwiesenweg 9
W-6370 Oberursel 4 (DE)**

(74) Vertreter : **Dr. Fuchs, Dr. Luderschmidt
Dipl.-Phys. Seids, Dr. Mehler Patentanwälte
Abraham-Lincoln-Strasse 7
W-6200 Wiesbaden (DE)**

# Beschreibung

Die Erfindung betrifft eine Vorrichtung zur Behandlung von Blut im extrakorporalen Kreislauf, insbesondere Hämodialysevorrichtung gemäß dem Oberbegriff des Anspruchs 1 und eine Hämofiltrationsvorrichtung gemäß dem Oberbegriff des Anspruchs 3.

Eine Hämodialysevorrichtung weist ein Austauschelement, insbesondere einen Dialysator auf, der durch eine Membran in zwei Kammern geteilt ist, wobei die erste Kammer in einen Dialysierflüssigkeitsweg und die zweite Kammer in einen Blutweg geschaltet ist, der Dialysierflüssigkeitsweg eine Dialysierflüssigkeitquelle aufweist, die wenigstens einen Konzentratzulauf, eine Frischwasserquelle und ein Temperiereinheit besitzt, der Blutweg wenigstens eine Blutpumpe und stromab des Dialysators eine Tropfkammer aufweist.

Eine Hämofiltrationsvorrichtung weist einen Hämofilter auf, das ebenfalls durch eine Membran in zwei Kammern geteilt ist, wobei die erste Kammer zur Aufnahme von mit Stoffwechselprodukten beaufschlagtem Hämofiltrat aus Blut dient und die zweite Kammer gleichfalls in einen Blutweg geschaltet ist. Dabei wird die entzogene Hämofiltratmenge in etwa durch eine gleiche Menge an Substitutionsflüssigkeit (abzüglich der zu ultrafiltrierenden Menge) ersetzt, die stromab des Austauschelements mit einer bestimmten Temperatur dem Blut zugesetzt wird.

Bei der Dialyse oder Hämofiltration eingesetzte Blutreinigungsgeräte sollen bekanntlich die funktionsunfähige Niere im Fall der akuten oder chronischen Urämie ersetzen. Obwohl diese Geräte noch nicht vollkommen sind, ist es jedoch im Laufe der Zeit gelungen, die Verfahren soweit zu entwickeln, daß einer großen Anzahl von chronisch urämischen Patienten ein Überleben bei befriedigender Lebensqualität gesichert werden kann.

Um zumindest die für die Lebenserhaltung notwendigen Funktionen der Niere zu ersetzen, muß das Blut von Urämietoxinen befreit werden, der Elektrolythaushalt und der Säure-Basehaushalt ausgeglichen und ein Teil der über Nahrungsmittel und Getränke aufgenommenen Flüssigkeitsmenge entfernt werden.

Überwiegend erfolgt dies mit dem Verfahren der Hämodialyse, bei dem Blut in einem extrakorporalen Kreislauf an einer semipermeablen Membran vorbeigeführt wird, an deren anderer Seite eine Dialysierflüssigkeit vorbeigeleitet wird, deren Elektrolytzusammensetzung im wesentlichen der des Blutes gleicht. Dabei treten die Urämietoxine vom Blut in die Dialysierflüssigkeit über, wodurch es zu einem Ausgleich der Konzentrationen kommt. Soweit eine Druckdifferenz zwischen Blut und der Dialysierflüssigkeit hergestellt wird, kommt es auch zu einem Übertritt von Flüssigkeit, also von Ultrafiltrat und damit zum gewünschten Flüssigkeitsentzug. Infolge der semipermeablen Eigenschaften der Membran werden dabei die lebenswichtigen Blutkörperchen und Proteine im Blut zurückgehalten.

Bei einem anderen bekannten Verfahren, nämlich der Hämofiltration, wird Blut in einem extrakorporalen Kreislauf durch ein Hämofilter geleitet, durch das ein Ultrafiltrat abgezogen wird, das Urämietoxine enthält. Zugleich wird eine in erster Nährung gleichgroße Menge einer urämietoxinfreien Substitutionslösung dem Blut wieder zudosiert. In dem in diesem Verfahren befindlichen Austauschelement, welches ein Dialysator bzw. ein Hämofilter sein kann, findet also ein Wechsel von Flüssigkeitsteilen in eine andere Flüssigkeit bzw. ein Vorbeigleiten einer Flüssigkeit an einer anderen Flüssigkeit, beispielsweise Blutplasma an Dialysierflüssigkeit, statt. Da diese Flüssigkeiten unterschiedliche Temperaturen aufweisen, erfolgt demzufolge auch ein Temperaturaustausch.

In der DE-OS 33 13 421 sind derartige Hämodialyse und Hämofiltrationseinrichtungen beschrieben. Bei diesen bekannten Vorrichtungen wird die Dialysierflüssigkeitsquelle bzw. Substituatquelle auf eine vorbestimmte Temperatur mit Hilfe einer Temperiereinheit erwärmt, wobei die Temperatur der Quelle von einem Temperaturdetektor an eine Regeleinheit abgegeben wird, die auf diesen gemessenen Temperaturwert und einen vorgegebenen Temperaturwert hin die Heizeinrichtung regelt. Dabei wird der vorgegebene Temperaturwert von dem behandelnden Arzt vor der Dialysesitzung eingegeben und anschließend während der gesamten Behandlung konstant gehalten.

In einem Artikel von Q. Maggiore u.a. in Proc. EDTA (1981) Vol.18, S. 597-602, wird gezeigt, daß die häufig beobachtete und zu diesem Zeitpunkt allgemein akzeptierte bessere Blutdruckstabilität bei dem Verfahren der isolierten Ultrafiltration darauf zurückzuführen ist, daß bei diesem Verfahren dem Blut des Patienten Wärmeenergie entzogen wird. Insbesondere zeigte es sich, daß auch während der konventionellen Hämodialyse eine bessere Blutdruckstabilität erzielt wird, wenn die Temperatur der Dialysierflüssigkeit von dem üblichen Wert um 37°C auf 34°C abgesenkt wird. Allerdings zeigte sich auch, daß manche Patienten diese Temperaturabsenkung als unangenehm empfanden.

Durch diese Maßnahme wurde die Bluttemperatur im arteriellen Schlauchsystem im wesentlichen konstant gehalten. Als Meßmittel dienten direkt in das Schlauchsystem eingesetzte Thermistoren. Deshalb mußte das gesamte System nachsterilisiert werden.

In einem späteren Artikel von Q. Maggiore u.a. in Trans Am Soc Artif Intern Organs (1982), Vol.28,S. 523-527, wird gezeigt, daß auch bei der Hämofiltration ein Einfluß der Wärmeenergiebilanz auf die Kreislaufstabilität besteht. Es wurden Messung durchgeführt, aus denen hervorgeht, daß bei der konventionellen Hamodialyse dem extrakorporalen Kreislauf Energie zugeführt wird. Darin wird eine Ursache für den Anstieg der Körpertemperatur gesehen, die sich nachteilig auf das Befinden des Patienten auswirkt. Als Meßmittel im extrakorporalen Kreislauf wurden spezielle Kunststoffeinmalartikel verwendet, die die Einführung eines Thermistors ohne direkten Blutkontakt erlaubten. Dabei taucht eine konstante Meßabweichung von 1°C auf.

Während K. Schäfer u.a. in The International Journal of Artificial Organs (1983), Vol.6, S. 75-76, einen Einfluß der Bluttemperatur und somit der Wärmeenergiebilanz auf die Kreislaufstabilität bezweifelt, bestätigen T. Kishimoto u.a. in Dialysis & Transplantation (1986) Vol.15, Nr.6, S. 329-333, die Auffassungen von Q. Maggiore, soweit sie auf die Hämodialyse bezogen waren.

Bekanntermaßen entsteht in der Regel auf der Rückflußseite bei der Hämodialyse bzw. auf der Zuflußund Rückflußseite im extrakorporalen Blutkreislauf bei der Hämofiltration ein Energieverlust.

Die fest eingegebene Senkung der Temperatur der Dialysierflüssigkeit reicht häufig nicht aus um einen Temperaturausgleich zu schaffen. Dies ist auf die Reaktion der einzelnen Patienten auf die jeweiligen Dialysierbedingungen zurückzuführen. Insofern kann also bei der bekannten Temperatursenkung nicht sichergestellt werden, daß der Patient ausreichend temperaturkompensiert wird, d.h. häufig tritt dennoch eine fiebrige Reaktion auf, die zu einer Verringerung des peripheren Widerstand des Patienten und somit zu einer erheblichen Belastung führt.

Es ist somit Aufgabe der Erfindung, die Vorrichtung der eingangs erwähnten Art so fortzubilden, daß die Energiebilanz eines der Hämodialyse oder Hämofiltration unterzogenen Patienten verbessert wird.

Erfindungsgemäß wird diese Aufgabe durch die kennzeichnenden Merkmale des Anspruchs 1 bzw. des Anspruchs 3 gelöst.

Erfindungsgemäß wird die Bluttemperatur, die mit der Körpertemperatur des Patienten korreliert, gemessen und mit einem vorbestimmten Wert bzw. einer Reihe von vorbestimmten Werten verglichen, wobei entsprechend dem Vergleichsergebnis die Temperatur der Dialysierflüssigkeit bzw. der Substitutionslösung eingestellt wird.

Ein zweiter Temperatursensor im venösen Teil des extrakorporalen Kreislaufes gestattet es, bei Kenntnis des Blutflusses, der i.d.R. gegeben ist, den Wärmeentzug, den der Patient erfährt, präzise zu bestimmen, ohne daß die Parameter des extrakorporalen Kreislaufes sowie die Dialysierflüssigkeits- bzw. Substituat- und Umgebungstemperatur näher bekannt sein müssen.

Die am Beginn der Dialyse oder der Hämofiltration gemessene arterielle Bluttemperatur und/oder venöse Bluttemperatur wird als Referenztemperatur vorgegeben. Eine weitere Referenzvorgabe kann sich ergeben aus der am Beginn der Dialyse gemessenen Differenz zwischen der arteriellen und der venösen Bluttemperatur. Weiterhin kann als Referenzwert eine bestimmte, zu erzielende Leistung vorgegeben werden.

Die Auswerte- und Regeleinheit ist also eingangsseitig sowohl mit der Blutpumpe und dem arteriellen Temperatursensor als auch gegebenenfalls mit dem venösen Temperatursensor verbunden. Ausgangsseitig ist die Auswerte- und Regeleinheit mit der Temperiereinheit der Dialysierflüssigkeitsquelle bzw. der Substituatquelle verbunden.

Über ein Eingabegerät werden die gewählten Referenzwerte der Auswerte- und Regeleinheit eingegeben.

Die Erfindung gestattet es, mit Hilfe der Messung der Bluttemperatur im arteriellen Blutweg des extrakorporalen Kreislaufes, den Wärmeentzug während dem Hämodialysevorgang, aber auch der Hämofiltration oder Oxygenie so zu beeinflussen, daß die Körpertemperatur konstant gehalten oder nach Wahl des Arztes auch definiert verändert werden kann.

Bei den in vivo Versuchen wurde bei der Hämodialyse Dialysierflüssigkeitstemperaturen von 37°C und 34°C eingestellt und die Körpertemperatur sowie Kreislaufparameter, wie z.B. der Blutdruck in mehrminütigen Abständen gemessen. Darüberhinaus wurden bei in vivo Experimenten die Wärmeverluste im extrakorporalen Kreislauf in Abhängigkeit von der Dialysierflüssigkeit sowie der Umgebungstemperatur und dem Blutfluß gemessen und gezeigt, daß diese drei Parameter die Wärme- bzw. Temperaturbilanz am extrakorporalen Kreislauf belasten.

Eigene Untersuchungen haben gezeigt, daß bei einer Dialysierflüssigkeitstemperatur von 37°C das Blut im extrakorporalen Kreislauf abgekühlt wird, sofern die Zimmertemperatur im normalen Bereich von 20-25°C liegt. Dennoch steigen Körper- und Bluttemperatur arteriell an. Diese Messungen geben Hinweise darauf, daß der Anstieg der arteriellen Bluttemperatur bzw. Körpertemperatur von der Biokompatibilität des verwendeten Hämodialysators abhängt. Die Untersuchungen haben weiterhin ergeben, daß abgesehen von den oben zitierten Einflüssen von Dialysierflüssigkeitstemperatur, Umgebungstemperatur und Blutfluß auch noch Konstruktion und Ausführung von Blutschlauchsystem und Hämodialysator sowie der Dialysierflüssigkeitsfluß die Wärmebilanz beeinflussen.

Aus den zitierten Literaturquellen geht hervor, daß die Dialysierflüssigkeitstemperatur einen Einfluß auf die Kreislaufstabilität hat. Aus den eigenen Messungen ist bekannt, daß der Anstieg der Körpertemperatur nicht durch Aufheizen des Blutes im extrakorporalen Kreislauf begründet ist. Es muß sich vielmehr um eine Reaktion des Organismus auf den Vorgang der Hämodialyse handeln, wobei ein Einfluß der Biokompatibilität der verwendeten Materialien vermutet wird.

Die Aussagen treffen in gleicher Weise auch auf die Hämofiltration zu.

Die zur Stabilisierung der Körpertemperatur zu entziehende Wärmemenge wird daher von patientenspezifischen Parametern wie Empfindlichkeit des Patienten aber auch Körpergewicht abhängig sein.

Die vom Patienten im extrakorporalen Kreislauf entzogene Wärmemenge hängt also von einer Reihe bereits erwähnter Parameter ab. Alle erwähnten Einflüsse sind i.d.R. nicht oder bestenfalls näherungsweise vorhergesagt worden.

An zwei Ausführungsbeispielen wird die Erfindung näher erläutert.

Es zeigen

Fig. 1 eine Vorrichtung zum Entziehen von Wärme aus Blut im extrakorporalen Kreislauf für die Hämodialyse und

Fig. 2 eine Hämofiltrationsvorrichtung zum gleichen Zweck für die Hämofiltration.

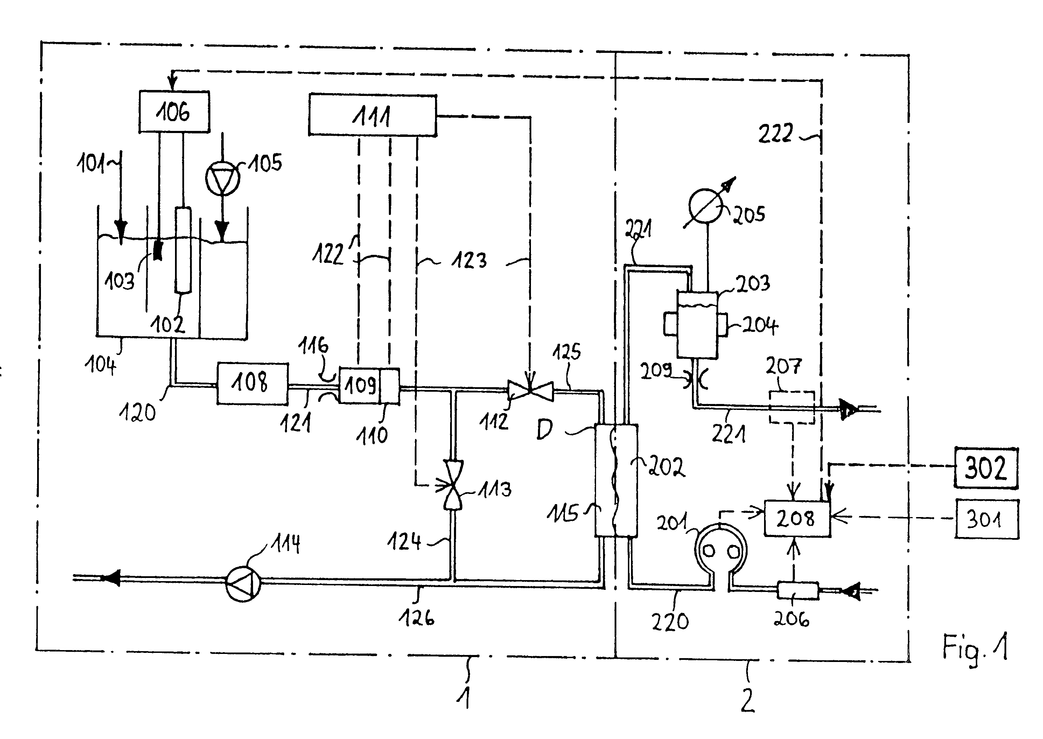

Das Hamodialysegerät gemäß Fig. 1 besteht dabei aus einem Dialysierflüssigkeitsteil 1 und dem extrakorporalen Blutkreislauf 2. Beide Teile haben einen im wesentlichen bekannten Aufbau. So besteht eine Dialysierflüssigkeitsquelle aus einem Wasserzulauf 101, dessen Quelle hier nicht näher dargestellt ist, aus einem elektrisch betriebenen Heizer 102 mit Temperatursensor 103 sowie einer Konzentrat fördernden Pumpe 105 und einem Dialysierflüssigkeitsbehälter 104. Der elektrisch betriebene Heizer 102 und der Temperatursensor 103 sind mit einer Regeleinheit 106 verbunden, die mit Hilfe eines Signals des Temperatursensors 103 den Heizer 102 so regelt, daß eine vorgegebene Temperatur der Dialysierflüssigkeit erreicht wird. Vom Dialysierflüssigkeitsbehälter 104 führt eine Leitung 120 zu einer an sich bekannten Entgasungseinheit 108. Diese wiederum ist verbunden mit einem Leitfähigkeitssensor 109 und einem dazugehörigen Temperaturssensor 110. Die Signale dieser Sensoren 109 und 110 werden von einem Überwachungsgerät 111 verarbeitet und daraus die temperaturskompensierte Leitfähigkeit der Dialysierflüssigkeit errechnet und diese sowie die Temperatur mit vorgegebenen Alarmgrenzwerten verglichen und bei Abweichungen außerhalb des vorgegebenen Alarmfensters die Ventile 112 und 113 so gesteuert, daß die Dialysierflüssigkeit am Dialysator vorbeigeleitet wird. Eine stromab des Dialysators D angeordnete Pumpe 114 dient der Förderung der Dialysierflüssigkeit und erzeugt in Verbindung mit einer stromauf des Dialysators D angeordneten Drossel 116 einen Unterdruck im Dialysierflüssigkeitskompartiment 115 des Dialysators D zum Zwecke der Ultrafiltration.

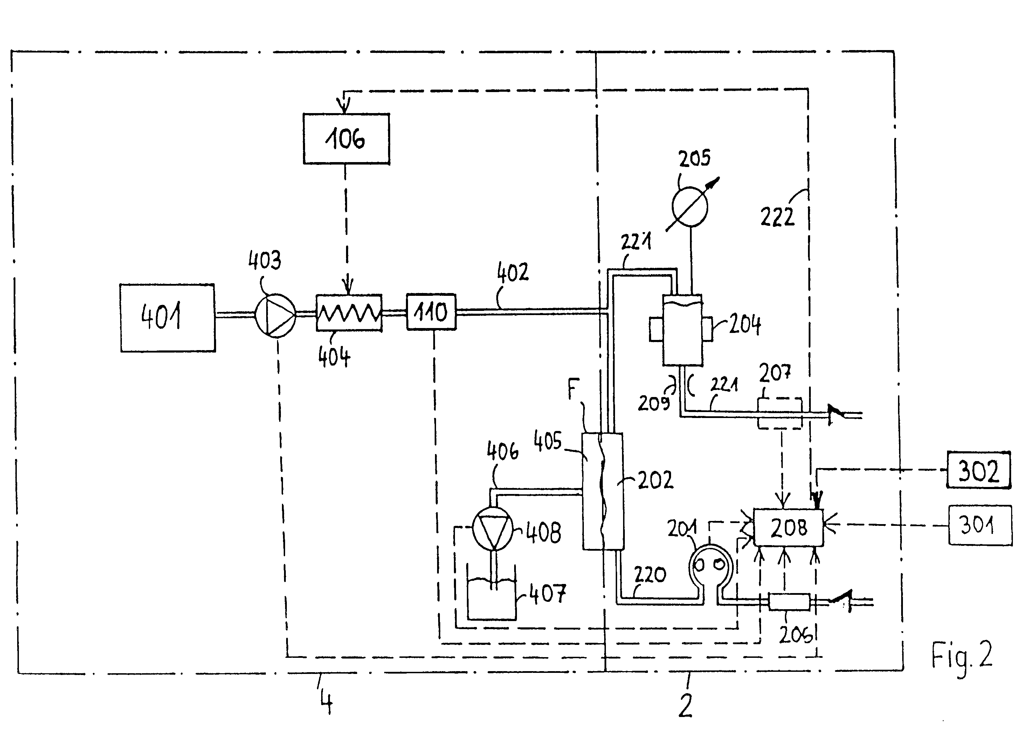

Das Hämofiltrationsgerät gemäß Fig. 2 besitzt anstelle des Dialysierflüssigkeitsteils 1 ein Substitutionslösungsteil 4 und ebenfalls einen extrakorporalen Kreislauf 2.

Auch das Substitutionslösungsteil 4 hat einen im wesentlichen bekannten Aufbau. So besteht dieses aus einem Substitutionsbehälter 401, dem in einer Leitung 402 die Substituatpumpe 403 folgt. Diese dient der Förderung der Substitutionslösung in das Schlauchstück 221 des venösen Teils des extrakorporalen Blutkreislaufs 2. Der Substituatpumpe 403 ist eine Heizeinrichtung 404 nachgeordnet, die die Substitutionslösung auf einen bestimmten Temperaturwert erwärmt.

Diese Heizeinrichtung 404 ist dazu mit der Regeleinheit 106 verbunden. Zwischen der Heizeinrichtung 404 und der Einmündung der Leitung 402 in das Schlauchstück 221 ist noch ein zweiter Temperatursensor 110 angeordnet, während der erste Temperatursensor 206 stromauf in den arteriellen Teil 220 des Blutschlauchsystems eingeschaltet ist.

Vom Filtratkompartiment 405 führt eine Leitung 406 zu einem Auffangbehälter 407 für das Hämofiltrat. Das Hamofiltrat, welches aus abgezogenem Plasmawasser einschließlich der Stoffwechselprodukte besteht, wird über die zwischengeschaltete Filtratpumpe 408 in den Auffangbehälter 407 gefördert. Dabei wird die Förderrate der Filtratpumpe 408 an die Auswerte- und Regeleinheit 208 signalisiert, mit der ebenfalls die Substituatpumpe 403 und der Temperatursensor 110 zur Signalisierung der Förderrate bzw. der Temperatur der erwärmten Substituatlösung verbunden sind.

Der extrakorporale Kreislauf 2 ist bei der Hämodialyse und der Hämofiltration im wesentlichen gleich und besteht stromauf des Dialysators D bzw. Filter F aus der Blutpumpe 201, dem Blutkompartiment 202 des Dialysators D bzw. des Filters F, der stromab des Dialysators D bzw. Filters F angeordneten Tropfkammer 203 mit zugehörigen Füllstandssensoren 204 sowie einem venösen Druckmonitor 205. Im arteriellen Blutweg ist im Bereich des Schlauchstückes 220 ein erster Temperatursensor 206 eingeschaltet. Im venösen Blutweg kann im Bereich des Schlauchstückes 221, welches von der Tropfkammer 203 zum Patienten zurückführt, ein weiterer Temperatursensor 207 angeordnet sein. Dabei werden die üblichen Temperatursensoren eingesetzt, beispielsweise Platinwiderstände mit einem Nominalwiderstand von 100 Ohm bei 0°C oder Thermistoren, beispielsweise NTC- oder PTC-Thermistoren.

Eine Auswerte- und Regeleinheit 208 empfängt die Signale der Temperatursensoren 206 und 207 sowie ggf. ein blutpumpenflußproportionales Signal von der Blutpumpe 201. Bei der Hämofiltration empfängt die Auswerte- und Regeleinheit 208 ggf. weiterhin ein Signal über die Förderrate des abgezogenen Ultrafiltrats von der Filtratpumpe 408 und das Temperatursignal vom Temperatursensor 110, sowie von der Substituatpumpe 403. Zusätzlich können optionale, patienten- bzw. behandlungsspezifische Steuerparameter durch das Eingabegerät 301 eingegeben werden. Von der Steuereinheit 208 führt eine Signalleitung zur Temperiereinheit 106, die eine Änderung der Dialysierflüssigkeitstemperatur bzw. der Temperatur der Substitutionslösung ermöglicht.

Die Temperatursensoren 206 und 207 sind vorteilhafterweise so konstruiert, daß keine speziellen Einmalartikel nötig sind. Hingegen wird ein Teil des Schlauchstückes 220 und 221 in ein das Schlauchstück mit den Sensor gemeinsam umschließendes Gehäuse eingelegt, das so ausgeführt und isoliert ist, daß der Temperatursensor auf etwa 0,1°C reproduzierbar die in dieser Stelle im Schlauchsystem herrschende Temperatur mißt.

Bei der Hämodialyse wird über den Wasserzulauf 101 Wasser in den Dialysierflüssigkeitsbehälter eingeleitet. Ebenso erfolgt eine Konzentratzufuhr über die Konzentratpumpe 105. Der Dialysierflüssigkeitsbehälter 104 dient im wesentlichen zur Erwärmung, Entgasung und Mischung der Dialysierflüssigkeit, wobei letztere vorteilhafterweise volumetrisch erfolgt. Mit Hilfe des elektrisch betriebene Heizers 102 und dem dazugehörigen Temperatursensor 103 wird das Wasser auf einen vorher bestimmten Wert, beispielsweise 34°C erwärmt.

Die in der Entgasungseinheit 108 befindliche, nicht näher dargestellte Pumpe saugt die Dialysierflüssigkeit über die Leitung 120 an und setzt die in der Dialysierflüssigkeit gelöste Luft frei. Diese Luft wird zusammen mit einem Teil der Dialysierflüssigkeit zur Mischkammer des Dialysierflüssigkeitsbehälters 104 zurücktransportiert und entweicht dort. Einzelheiten hierzu wurden in der Zeichnung vernachlässigt, da diese allgemein bekannt sind. Die entgaste Dialysierflüssigkeit wird zunächst mit einem Leitfähigkeitssensor 109 und einem zugehörigen Temperatursensor 110 über Leitung 121 in Berührung gebracht. Das vorgeschaltete Drosselventil 116 hat den Zweck, den Unterdruck in der Dialysierflüssigkeit in Abhängigkeit von der Wirkung der Dialysierflüssigkeitspumpe zu erzeugen.

Die Sensoren 109 und 110 sind mit einem Überwachungsgerät 111 mittels Signalleitung 122 verbunden, welches im Fall eines Abweichens der Dialysierflüssigkeit von den eingestellten Werten Alarm gibt und dabei über die Signalleitung 123 die Ventile 112 und 113 steuert. So wird beim Abweichen der eingestellten Werte das Ventil 112 geschlossen und das Ventil 113 der Bypassleitung 124 geöffnet und dabei verhindert, daß Dialysierflüssigkeit unerwünschter Zusammensetzung oder Temperatur zum Dialysator D gelangt, dessen Eingang mit der Leitung 125 vom Ventil 112 verbunden ist. Die Dialysierflüssigkeit wird demzufolge über das Ventil 113 direkt zur Pumpe 114 gefördert.

Stromab des Dialysators führt eine Leitung 126 zur Pumpe 114 und ist dabei stromab mit der Bypassleitung 124 verbunden.

Bei der Hämofiltration wird der Substituatbehälter 401 an die Leitung 402 angeschlossen. Bei Inbetriebnahme der Substituatpumpe 403 wird die Substitionslösung über die Leitung 402 zur Heizeinrichtung 404 gefördert und dort auf einen vorbestimmten Temperaturwert erwärmt. Der Temperatursensor 110 mißt die Temperatur der Substitutionslösung und signalisiert diese zur Auswerte und Regeleinheit 208, währen die Förderrate von der Substituatpumpe 403 ebenfalls an die Einheit 208 abgegeben wird.

Der extrakorporale Blutkreislauf 2 erstreckt sich über das arterielle Schlauchstück 220 und die Blutpumpe 201 zum Blutkompartiment 202 des Dialysators D bzw. Filters F und über das venöse Schlauchstück 221, in die die Tropfkammer 203 eingeschaltet ist, zum Patienten. Der vordere Teil des Schlauchstückes 221 ist an seinem einen Ende mit dem Dialysator D bzw. dem Filter F und an seinem anderen Ende mit dem Eingang der Tropfkammer 203 verbunden.

Zur Förderung des Blutes dient die Blutpumpe 201, vorzugsweise eine Schlauchpumpe, die das Blut entsprechend der gewünschten Förderrate durch den extrakorporalen Kreislauf 2 fördert.

Die Tropfkammer 203 ist mit einem Füllstandssensor 204 verbunden, der beim Absinken des Niveaus in der Tropfkammer 203 bzw. beim Auftreten von Luft oder Blutschaum Alarm gibt, die Blutpumpe 201 stoppt und Drossel 209 schließt, die stromab der Tropfkammer 203 angeordnet ist.

Der venöse Druckmonitor 205 zeigt den venösen Rücklaufdruck an.

Ist im extrakorporalen Blutkreislauf nur ein Temperatursensor 206 angeordnet, so wird dessen arterielle Bluttemperatur als Referenztemperatur der Auswerte- und Regeleinheit 208 zugrunde gelegt. Ist zusätzlich ein Temperatursensor 207 im venösen Blutweg des extrakorporalen Kreislaufes angeordnet, so kann auch die Differenz zwischen arteriellem Temperatursensor 206 und venösem Temperatursensor 207 als Referenz für die Regelung gewählt werden.

Durch die Auswerte- und Steuereinheit 208 werden die Signale der Temperatursensoren 206 und 207 sowie ein blutpumpenflußproportionales Signal von der Blutpumpe 201 empfangen. Von der Auswerte- und Regeleinheit 208 führt nun eine Signalleitung 222 zur Temperiereinheit 106, die eine Verstellung der Temperaturregelung ermöglicht. Dadurch wird die Dialysierflüssigkeit bzw. Substitutionslösung auf einen von der Auswerte- und Regeleinheit 208 errechneten Temperaturwert gehoben bzw. abgesenkt. In der Folge wird entsprechend temperierte Dialysierflüssigkeit dem Dialysator D bzw. Substitutionslösung der venösen Blutleitung 221 zugeführt. Durch das Vorbeiströmen der beiden Medien Dialysierflüssigkeit und Blutplasma findet im Dialysator eine Wärmeaustausch statt. Die geschieht bei der Hämofiltration beim Einströmen der Substitutionslösung in die Blutleitung 221. In der Regel findet ein Austausch von Wärmeenergie zum Blut von der Dialysierflüssigkeit bzw. der Substitutionslösung statt. Die Körpertemperatur des Patienten bzw. dessen Bluttemperatur wird auf einen normalen bzw. von Arzt gewählten Wert gebracht.

Anschließend verliert das so aufgewärmte Blut beim Rücklauf zum Patienten wieder Energie – ca. 2°C –, so daß es den Patienten wieder im abgekühlten Zustand erreicht, was bei der Ermittlung der Energiebilanz berücksichtigt wird.

Bei der Hamofiltration wird ein Blutwärmeenergiewert $E_1$ durch die Erfassung des Temperaturwertes $T_1$ vom Temperatursensor 206 und des Fördervolumens $V_1$ der Blutpumpe 201 bestimmt

$$E_1 = T_1 \times V_1$$

Aus dem Temperaturverlust und dem Abziehen des Hämofiltrats ergibt sich ausgangsseitig am Hämofilter F im extrakorporalen Kreislauf 2 ein Energiewert

$$E_2 = T_2 \times (V_1 - V_2)$$

wobei $T_2$ empirisch bekannt ist und $V_2$ das abgezogene Hämofiltratvolumen darstellt. Dabei erreicht $T_2$ seinen Wert an der Stelle der Einmündung der Substituatleitung 402 in die venöse Blutleitung 221 des extrakorporalen Blutkreislaufes 2.

Der Energiewert $E_3$ ergibt sich aus dem gemessenen Temperaturwert $T_3$ des Temperatursensors 110 und dem Fördervolumen $V_3$ der Substituatpumpe 403

$$E_3 = T_3 \times V_3$$

Energiewert $E_4$ stellt sich dar aus dem vom Temperatursensor 207 gemessenen Temperaturwert $T_4$ und dem Fördervolumen $V_1$ der Blutpumpe 201 abzüglich der Ultrafiltrationsrate UF

$$E_4 = T_4 \times (V_1 - UF)$$

wobei

$$UF = V_2 - V_3$$

ist.

Da die Werte von $T_1$, $T_2$, $T_4$ sowie $V_1$, $V_2$, $V_3$ und damit auch UF bekannt sind bzw. in die Auswerte- und Regeleinheit 208 eingegeben werden, findet in dieser Einheit eine Energiebilanz statt, infolge derer der Temperaturwert $T_3$ für die zugeförderte Substitutionslösung ermittelt wird. Dadurch wird die Heizeinrichtung 404 durch die Regeleinheit 106, die ein entsprechendes Signal von der Auswerte- und Regeleinheit 208 erhält, solange in Betrieb gesetzt, bis die Temperatur der Substitutionslösung den Sollwert $T_3$ erreicht hat, der von der Auswerte- und Regeleinheit 208 errechnet wurde.

Da sich der Energiewert $E_4 = E_2 + E_3$ aus

$$E_4 = T_2 (V_1 - V_2) + V_3 \times T_3$$

errechnen läßt, kann ein Messen des Temperaturwertes $T_4$ entfallen, so daß der Temperatursensor 207 weggelassen werden kann.

Es kann jedoch auch der gemessene Temperaturwert des Temperatursensors 207 als Sollwert vorbestimmt und in die Auswerte- und Regeleinheit 208 eingegeben werden, wobei dieser mit dem gemessenen Wert des Temperatursensors 206 verglichen wird und sich aus dieser Temperaturdifferenz der Temperatur-Istwert des Temperatursensors 110 ergibt.

Erfindungswesentlich ist dabei, daß der am extrakorporalen Bluteingang, d.h. am Temperatursensor 206 ermittelte Temperaturwert mit dem am Blutausgang, d.h. am Temperatursensor 207 ermittelten Temperaturwert korreliert ist. Denn je höher der Eingangswert ist, desto tiefer ist der Ausgangswert einzustellen, d.h. desto weniger Energie ist dem Blut extrakorporal zuzuführen, da der Körper intrakorporal durch Erzeugen von Fieber zusätzliche Energie bereitstellt, die dem Körper des Patienten extrakorporal entzogen werden muß. Dies kann bei der Hämofiltration auf die vorstehend beschriebene Weise in zwei Ausführungsformen erfolgen, wobei eine ausgewogene Energiebilanz erreicht wird.

Der Abstand vom Patientenanschluß bis zu dem Temperatursensoren 206 bzw. 207 beträgt ca. 1 m. Messungen haben gezeigt, daß bei üblichen Raumtemperaturen zwischen 20 und 25°C und den üblichen Schlauchmaterialien der Temperaturabfall auf dieser Strecke 0,4°C beträgt, was durch geeignete Kalibrierung der Auswerte- und Regeleinheit 208 kompensiert werden kann.

Mit Hilfe der Eingabeeinheit 301 kann die Auswerte- und Regeleinheit 208 programmiert werden. Dabei werden folgende Regelmöglichkeiten vorteilhafterweise vorgesehen :

1. Der vom arteriellen Temperatursensor 206 am Beginn der Behandlung gemessene Messwert wird als Referenztemperatur gewählt.

2. Die vom Arzt einzustellende arterielle Bluttemperatur wird als Referenztemperatur gewählt.

3. Die Differenz zwischen arteriellem Temperatursensor 206 und venösem Temperatursensor 207 wird als Referenz für die Regelung gewählt.

4. Eine bestimmte, zu entziehende Leistung kann vorgewählt werden.

In einer zusätzlichen Anzeigeeinheit, die in der Zeichnung mit 302 bezeichnet ist, kann u.a. angezeigt werden :

– Die Bluttemperatur arteriell ;

– die Bluttemperatur venös ;

– die entzogene Leistung ;

– die vom Beginn der Behandlung an entzogene Energie bzw. Wärmemenge.

Daraus kann in der Anzeigeeinheit die Energiebilanz errechnet und angezeigt werden. Darüberhinaus können noch Einstellmöglichkeiten für die Einstellung von Regelzeitkonstanten vorgesehen werden, sofern nicht ein geeigneter adaptiver Regler auf Mikroprozessorbasis eingesetzt wird.

Selbstverständlich können andere Formen der Eingabe als die gezeigte Verwendung finden.

Wie bereits vorstehend erwähnt, können mit der Anzeigeeinheit 302 die vom Sensor 206 gemessene arterielle Bluttemperatur, die vom Sensor 207 gemessene venöse Bluttemperatur und bei Kenntnis der im externen Blutkreislauf geförderten Volumina $V_1$-$V_4$ die momentan dem Blut im Austauschelement F entzogene Energie sowie bei Integration über ein bestimmtes Zeitintervall, beispielsweise die gesamte Behandlungsdauer, die entzogene Leistung angezeigt werden.

Gemäß einer weiteren Ausführungsform kann die Auswerteeinheit 208 in Verbindung mit der Eingabeeinheit 301 und der Anzeigeeinheit 302 ein selbstständiges Gerät darstellen, daß mit bereits bestehenden Hämodialysegeräten oder Hämofiltrationsgeräten kombiniert wird. Hier muß lediglich dafür Sorge getragen werden, daß die entsprechenden Schlauchsysteme für den extrakorporalen Blutkreislauf die Temperatursensoren 206 und 207 aufweisen bzw. die Temperatursensoren 206 und 207, die bereits an der Auswerteeinheit 208 angeschlossen sind, mit den Schlauchsystemen verbunden werden können. Des weiteren muß in diesem Fall an der Regeleinheit 106 ein Anschluß für die Signalleitung 222 zur Eingabe des Temperatur-Soll-Werts vorgesehen sein.

**Ansprüche**

1. Vorrichtung zum Behandeln von Blut im extrakorporalen Kreislauf, aufweisend ein Austauschelement (D), das durch eine Membran in zwei Kammern (115, 202) geteilt ist, von denen die erste Kammer (115) in einen Behandlungsflüssigkeitsweg geschaltet ist, der mit einer Behandlungsflüssigkeitsquelle (101) verbunden ist, die mit einer Temperiereinheit verbunden ist, die einen in die Behandlungsflüssigkeitsquelle (101) eintauchenden Temperaturdetektor (103), eine Heizeinrichtung (102) und eine erste Regeleinheit (106), die durch Vergleich des vom Temperaturdetektor (103) ermittelten Ist-Werts mit einem Soll-Wert die Heizeinrichtung (102) regelt, aufweist, und von denen die zweite Kammer (202) in einen Blutweg geschaltet ist, der eine Blutpumpe (201) aufweist, dadurch gekennzeichnet, daß ein erster Temperatursensor (206) stromauf des Austauschelementes (D) im Blutweg angeordnet und mit einer zweiten Regeleinheit (208) verbunden ist, die mit der ersten Regeleinheit (106) verbunden ist und den vom ersten Temperatursensor (206) gemessenen Ist-Wert mit einem Soll-Wert vergleicht und in Abhängigkeit vom Vergleichsergebnis der ersten Regeleinheit (106) ihren Sollwert vorgibt.

2. Vorrichtung nach Anspruch 1, dadurch gekennzeichnet, daß das Austauschelement (D) ein Hämodialysator bzw. ein Hämodiafilter und die Behandlungsflüssigkeit eine Dialysierflüssigkeit ist.

3. Vorrichtung zum Behandeln von Blut im extrakorporalen Kreislauf, aufweisend ein Hämofilter (F), das durch eine Membran in zwei Kammern (405, 202) geteilt ist, von denen die erste Kammer (405) zur Aufnahme von Hämofiltrat aus Blut dient und die zweite Kammer (202) in einen Blutweg geschaltet ist, eine stromauf des Hämofilters (F) angeordnete Blutpumpe (201) sowie eine Substitutionslösung zur Verfügung stellende Einrichtung (401), die über eine Substituatleitung (402) mit dem Blutweg verbunden ist, in die eine Substituatpumpe (403) und eine Temperiereinheit, die eine Heizeinrichtung (404) und eine erste die Heizeinrichtung (404) durch Soll-Ist-Wertvergleich regelnde Regeleinheit (106) aufweist, eingeschaltet sind, dadurch gekennzeichnet, daß ein erster Temperatursensor (206) stromauf des Hämofilters (F) im Blutweg angeordnet und mit einer zweiten Regeleinheit (208) verbunden ist, die mit der ersten Regeleinheit (106) verbunden ist, die den vom Temperatursensor (206) gemessenen Ist-Wert mit einem Soll-Wert vergleicht und in Abhängigkeit vom Vergleichsergebnis der ersten Regeleinheit (106) ihren Sollwert vorgibt.

4. Vorrichtung nach einem der Ansprüche 1, 2 oder 3, dadurch gekennzeichnet, daß ein zweiter Temperatursensor (207) stromab des Austauschelementes (D) bzw. des Hämofilters (F) im Blutweg angeordnet und mit der zweiten Regeleinheit (208) verbunden ist.

5. Vorrichtung nach einem der Ansprüche 1 bis 4, dadurch gekennzeichnet, daß die zweite Regeleinheit (208) mit einem programmierbaren Eingabegerät (301) gekoppelt ist.

6. Vorrichtung nach einem der Ansprüche 1 bis 5, dadurch gekennzeichnet, daß die zweite Regeleinheit (208) aus dem Ist-Wert, dem Soll-Wert, sowie aus der Pumprate der Blutpumpe (201) den dem Blut zu entziehenden Wärmestrom ermittelt und in Abhängigkeit davon der ersten Regeleinheit (106) ihren Soll-Wert vorgibt.

7. Vorrichtung nach Anspruch 6, dadurch gekennzeichnet, daß eine Anzeigeeinheit (302) die von dem ersten und dem zweiten Temperatursensor (206 ; 207) gemessenen Temperaturwerte und die aus diesen Temperaturwerten und aus der Pumprate der Blutpumpe (201) errechneten Wärmeströme anzeigt.

8. Blutreinigungsvorrichtung mit einem extrakorporalen Blutkreislauf (2), in den ein Austauschelement (D) oder ein Hämofilter (F) geschaltet ist und der eine eine bestimmte Förderrate fördernde Blutpumpe (201) aufweist, einem ersten Temperatursensor (206) zum Anordnen stromauf des Austauschelementes (D) bzw. des Hämofilters (F) und einem zweiten Temperatursensor (207) zum Anordnen stromab des Austauschelementes (D) bzw. des Hämofilters (F) gekennzeichnet durch eine Auswerteeinheit (208), die mit den Temperatursensoren (206, 207) sowie mit der Blutpumpe (201) verbunden ist und die aus der Differenz der fortlaufend von den Temperatursensoren (206, 207) ermittelten Temperaturwerte und der Förderrate der Blutpumpe (201) den Wärmestrom ermittelt.

9. Blutreinigungsvorrichtung mit einem extrakorporalen Blutkreislauf (2), in den ein Austauschelement (D) oder ein Hämofilter (F) geschaltet ist und der eine eine bestimmte Förderrate fördernde Blutpumpe (201) aufweist, und einem ersten Temperatursensor (206) zum Anordnen stromauf des Austauschelementes (D) bzw. des Hämofilters (F) gekennzeichnet durch eine Auswerteeinheit (208), die mit dem ersten Temperatursensor (206) und der Blutpumpe (201) verbunden ist und die aus dem zu Beginn der Blutreinigungsbehandlung mit dem Temperatursensor (206) gemessenen Temperaturwert sowie den zeitlich darauf folgenden Temperaturwerten jeweils Differenzwerte bildet und aus den fortlaufend gebildeten Differenzwerten und der Förderrate der Blutpumpe (201) in zeitlicher Folge den Wärmestrom ermittelt.

**Claims**

1. An apparatus for treating blood in an extracorporeal circuit comprising an exchange element (D) which is divided by a membrane into two chambers (115, 202), the first chamber (115) being connected in a treatment solution path with a treatment solution source (101), said treatment solution source being coupled to a temperature regulating unit having a temperature detector (103) immersing into said treatment solution source (101), further comprising a heating means (102) and a first control unit (106), which controls said heating means (102) by comparison of the actual value detected by said temperature detector (103) with a preselected desired value, said second chamber (202) being connected in a blood path with a blood pump (201), characterized in that a first temperature sensor (206) is disposed in said blood path upstream of said exchange element (D) and coupled to a second control unit (208) which itself is coupled to said first control unit (106) and which compares the actual value measured by said first temperature sensor (206) with a preselected desired value and sets in dependance from the result of the comparison the preselected desired value for said first control unit (106).

2. The apparatus according to claim 1, characterized in that said exchange element (D) is a hemodialyzer or a hemofilter, respectively, and that the treatment solution is a dialysis solution.

3. Apparatus for treating blood in an extracorporeal circuit comprising a hemofilter (F) which is divided by a membrane into two chambers (405, 202), the first chamber (405) serving the reception of hemofiltrate from blood and the second chamber (202) being connected in a blood path, further comprising a blood pump (201) being disposed upstream of said hemofilter (F) as well as a substitution fluid supply unit (401) coupled to said blood path through a substituate conduit (402) into which conduit a substituate pump (403) and a temperature regulating unit are connected, said temperature regulating unit comprising a first control unit (106) controlling the heating means (404) through a comparison of actual and preselected desired value, characterized in that a first temperature sensor (206) is disposed in said blood path upstream of said hemofilter (F) and coupled to a second control unit (208) which is coupled to said first control unit (106) and which compares the actual value measured by said temperature seonsor (206) with a preselected desired value and sets in dependance from the result of the comparison the preselected desired value for said first control unit (106).

4. Apparatus according to any of claims 1, 2 or 3, characterized in that a second temperature sensor (207) is disposed in said blood path downstream of said exchange element (D) or the hemofilter (F), respectively, and is coupled to said second control unit (208).

5. Apparatus according to any of claims 1 to 4, characterized in that said second control unit (208) is coupled to a programmable input unit (301).

6. Apparatus according to any of claims 1 to 5, characterized in that said second control unit (208) ascertains the heat flow to be withdrawn from the blood from the actual value as well as from the pump rate of said blood pump (201) and sets in dependance therefrom the preselected desired value for said first control unit (106).

7. Apparatus according to claim 6, characterized in that a display unit (302) displays the temperature values measured by said first and said second temperature sensor (206 ; 207) and further displays the heat flows calculated from said temperature values and from the pump rate of said blood pump (201).

8. A blood cleaning apparatus having an extracorporeal circuit (2) in which an exchange element (D) or a hemofilter (F) is connected and which comprises a blood pump (201) supplying a defined supply rate, further comprising a first temperature sensor (206) disposed upstream of said exchange element (D) or said hemofilter (F), respectively, and further comprising a second temperature sensor (207) disposed downstream of said exchange element (D) or said hemofilter (F), respectively, characterized by an evaluating unit (208) which is coupled to said temperature sensors (206, 207) as well as said blood pump (201) and which ascertains the heat flow from the difference of the temperature values, continuously determined from said temperature sensors (206, 207), and the pump rate of said blood pump (201).

9. A blood cleaning apparatus having an extracorporeal circuit (2) in which an exchange element (D) or a hemofilter (F) is connected and which comprises a blood pump (201) supplying a defined supply rate, further comprising a first temperature sensor (206) disposed upstream of said exchange element (D) or said hemofilter (F), respectively, characterized by an evaluating unit (208) which is coupled to said first temperature sensor (206) and said blood pump (201) and which forms difference values from the temperature value measured by said temperature sensor (206) at the beginning of the blood cleaning treatment as well as the temporally subsequent temperature values and which determines the heat flow from the continuously formed difference values and the pump rate of said blood pump (201) in temporal sequence.

**Revendications**

1. Dispositif pour le traitement du sang dans un circuit extracorporel, comportant un élément échangeur (D), qui est subdivisé par une membrane en deux chambres (115, 202), dont la première (115) est insérée dans le trajet du liquide de traitement, qui est raccordé à une source (101) du liquide de traitement raccordée à une unité de mise en température qui possède un détecteur de température (103) immergé dans la source (101) du liquide de traitement, un dispositif de chauffage (102) et une première unité de régulation (106) qui règle le dispositif de chauffage (102) par comparaison de la valeur réelle déterminée par le détecteur de température (103) à une valeur de consigne, tandis que la seconde chambre (202) est insérée dans un trajet de circulation du sang, qui comporte une pompe à sang (201),
caractérisé par le fait
qu'un premier capteur de température (206) est disposé dans le trajet de circulation du sang en amont de l'élément échangeur (D) et est raccordé à une seconde unité de régulation (202), qui est reliée à la première unité de régulation (106) et compare la valeur réelle mesurée par le premier capteur de température (206) à une valeur de consigne et prédétermine la valeur de consigne pour la première unité de régulation (106), en fonction du résultat de la comparaison.

2. Dispositif selon la revendication 1, caractérisé en ce que l'élément échangeur (D) est un hémodialyseur ou un filtre d'hémodialyse et le liquide de traitement est un liquide de dialyse.

3. Dispositif pour le traitement du sang dans un circuit extracorporel comportant un hémofiltre (F), qui est subdivisé par une membrane en deux chambres (405, 202), dont la première (405) sert à loger l'hémofiltrat tiré du sang et dont la seconde (202) est insérée dans un trajet de circulation du sang, une pompe à sang (201) disposée en amont de l'hémofiltre (F), ainsi qu'un dispositif (401), qui délivre une solution de substitution et est raccordé au trajet de circulation du sang par l'intermédiaire d'une canalisation (402) véhiculant le résultat de substitution et dans laquelle sont insérées une pompe (403) comprenant le résultat de substitution et une unité de mise en température, qui possède un dispositif de chauffage (404) et une première unité de régulation (106), qui règle le dispositif de chauffage (404) au moyen d'une comparaison valeur de consigne-valeur réelle,
caractérisé par le fait
qu'un premier capteur de température (206) est disposé dans le trajet de circulation du sang en amont de l'hémofiltre (F), et est raccordé à une seconde unité de régulation (408), qui est reliée à la première unité de régulation (106) qui compare la valeur réelle mesurée par le capteur de température (206) à une valeur de consigne et prédétermine la valeur de consigne de la première unité de régulation (106) en fonction du résultat de la comparaison.

4. Dispositif selon l'une des revendications 1, 2 ou 3, caractérisé en ce qu'un second capteur de température (207) est installé dans le trajet de circulation du sang en aval de l'élément échangeur (D) ou de l'hémofiltre (F) et est relié à la seconde unité de régulation (208).

5. Dispositif suivant l'une des revendications 1 à 4, caractérisé par le fait que la seconde unité de régulation (208) est accouplée à un appareil d'introduction programmable (301).

6. Dispositif suivant l'une des revendications 1 à 5, caractérisé par le fait que la seconde unité de régulation (208) détermine, à partir de la valeur réelle, de la valeur de consigne et du débit de pompage de la pompe à sang (201), le flux thermique devant être prélevé du sang et prédétermine, en fonction de cela, la valeur de consigne pour la première unité de régulation (106).

7. Dispositif selon la revendication 6, caractérisé en ce qu'une unité d'affichage (302) indique les valeurs de température mesurées par les premier et second capteurs de température (206 ; 207) et les flux 'thermiques calculés à partir de ces valeurs de température à partir du débit de pompage de la pompe à sang (201).

8. Dispositif d'épuration du sang comportant un circuit extracorporel de circulation du sang (2), dans lequel est inséré un élément échangeur (D) ou un hémofiltre (F) et qui comporte une pompe à sang (201) présentant un débit d'entraînement déterminé, et comportant un premier capteur de température (206) destiné à être disposé en amont de l'élément échangeur (D) ou de l'hémofiltre (F) et un second capteur de température (207) destiné à être disposé en aval de l'élément échangeur (D) ou de l'hémofiltre (F), caractérisé par une unité d'évaluation (208), qui est raccordée aux capteurs de température (206, 207) ainsi qu'à la pompe à sang (201) qui détermine le flux thermique à partir de la différence des valeurs de température déterminées en permanence par les capteurs de température (206, 207), et à partir du débit de la pompe à sang (201).

9. Dispositif d'épuration du sang comportant un circuit extracorporel de circulation du sang (2), dans lequel est inséré un élément échangeur (D) ou un hémofiltre (F) et qui comporte une pompe à sang (201) présentant un débit d'entraînement déterminé, et comportant un premier capteur de température (206) destiné à être disposé en amont de l'élément échangeur (D) ou de l'hémofiltre (F), caractérisé par une unité d'évaluation (208), qui est reliée au premier capteur de température (206) et la pompe à sang (201) et forme respectivement des valeurs de différence à partir de la valeur de température, mesurée au début du traitement d'épuration du sang par le capteur de température (206), et des valeurs de température qui succèdent dans le temps à cette valeur de température, et détermine selon une séquence temporelle le flux thermique à partir des valeurs de différence formées en continu et à partir du débit de refoulement de la pompe à sang (201).

Fig. 1

EP 0 265 795 B1

Fig. 2